# EUROPEAN PATENT APPLICATION

(11) **EP 1 867 357 A1**
(43) Date of publication of application: **19.12.2007**
(21) Application number: 06012088.8
(22) Date of filing: 13.06.2006
(51) Int. Cl.: A61M 15/06, A24F 47/00

(54) **Inhaler**

(71) Applicant: TrendTech A/S, 8620 Kjellerup (DK)
(72) Inventor: Jensen, Anders Leonhard, 8620 Kjellerup (DK)
(74) Representative: Sundien, Thomas

(57) **Abstract**

This invention relates to an inhaler (101) for the administration of one or more substances (106,108) to the human or animal body by means of inhalation, the inhaler (101) comprising at least two modules (105,103) arranged in extension of one another of which one module is a compartment module (105) comprising one or more compartments (107) with substance(s) (106,108), and one module is a mouthpiece module (103) comprising a mouthpiece (103) with means for perforating said compartments (107) in said compartment modules (105) thereby allowing said substance(s) (106,108) to be inhaled through the mouthpiece (103). The inhaler (101) may further comprise a heating module (104) with a heating means (104) for heating the inhaled air.
The invention further relates to a mouthpiece module, a compartment module and to a heating module.

## Description

The present invention relates to an inhaler for the administration of one or more substances to the human or animal body by means of inhalation. The invention further relates to a mouthpiece module, a compartment module and to a heating module for use in an inhaler.

### BACKGROUND

Tobacco use represents a considerable health hazard, but many smokers are nevertheless unwilling or unable to stop smoking without help. Apart from the health hazard to the smoker him or herself, the smoke from cigarettes, pipes, etc. also causes non negligible discomfort and even smoke related diseases to persons nearby (passive smoking) as well as to the environment. Among the many hurdles which smokers trying to quit have to overcome is the discomfort associated with tobacco withdrawal and urges to smoke.

Medications are now available which alleviate at least some of these problems, including nicotine replacement treatments. Their efficacy, however, remain relatively limited and there is a need for further improvements. Moreover, it has been observed that pure nicotine in the doses that smokers receive is relatively safe while the other constituents of tobacco smoke (tar and noxious gases) are primarily responsible for the health risks from smoking. Hence a pure nicotine delivery device could be used as an aid to smoking cessation and as a means of reducing withdrawal discomfort, and as a safer alternative to smoking also removing effectively the harmful effects of passive smoking.

There are now many different forms of pure nicotine delivery available. These include nicotine patches, nicotine chewing gums, nicotine tablets, nicotine nasal spray, nicotine solution capable of being added to beverages and different types of nicotine inhalers. All of these methods have strengths and weaknesses as delivery systems.

One major obstacle they face is that they deliver nicotine slowly relative to cigarettes. Nicotine from cigarette smoke is absorbed via the lungs and so reaches the brain within a few seconds of each puff. It has been noted that this fingertip control of smoking dose is important in maintaining smoking because individuals differ in their particular needs with regard to nicotine dose and aim to adjust their dose to meet those needs.

Inhalators come the closest to a cigarette in providing this fingertip control, but in existing inhalers the nicotine delivery is slow and inefficient because of the design of the delivery system involving sucking the vapour through a narrow bore tube akin to a cigarette holder which means that the passage of nicotine into the upper airways is impaired by the high draw resistance and narrow bore of the tip of the device. Further, inhalers known in the art (such as from WO 2004/098324) have the disadvantage of only having a rather limited time of durability due to the evaporation of the nicotine and aromatic substances. Also existing inhalers are of designs which make them relatively expensive to produce and manufacture whereby they do not constitute a cheaper alternative to normal cigarettes.

### OBJECT AND SUMMARY OF THE INVENTION

It is therefore an object of the present invention to provide an inhaler or parts for an inhaler which overcome some or all of the above-mentioned problems.

According to one aspect, the present invention relates to an inhaler for the administration of one or more substances to the human or animal body by means of inhalation, the inhaler comprising at least two modules arranged in extension of one another of which
- one module is a compartment module comprising one or more compartments with substance(s), and
- one module is a mouthpiece module comprising a mouthpiece with means for perforating said compartments in said compartment modules thereby allowing said substance(s) to be inhaled through the mouthpiece.

In an embodiment one module is a heating module comprising heating means for heating the air to be inhaled through said mouthpiece of said mouthpiece module.

In an embodiment a compartment in one compartment module is closed by the module being in extension of said compartment module.

In an embodiment said substances comprise nicotine and one or more aroma substances.

In an embodiment the compartment module comprises two separate compartments.

In an embodiment the compartment module comprises end faces arching inwards towards the interior of the compartment.

In an embodiment the compartment module comprises end faces with imprint patterns for facilitating perforation of the end faces, thereby the compartments.

In an embodiment the perforation means of said mouthpiece module comprise a hollow piston.

In an embodiment the mouthpiece module further comprises a filter.

In an embodiment said heating means are made of a material which has good heat-convecting or heat-conducting properties, such as cupper, brass or bronze adapted to be heated by an external heat source.

In an embodiment said heating means are pin shaped.

In an embodiment said heating means are shaped like a hollow cylinder adapted to receive a pin shaped heating source.

In an embodiment the heating means are made from foamed metal.

In an embodiment the heating means comprise a heat indicator.

In an embodiment the heating module comprises isolation means for heat isolating the heating means from other modules of the inhaler.

In an embodiment the heating module comprises a detachable cover for covering the heating means.

In an embodiment the heating means are chamber and at least partly filled with an accumulating material having good heat absorbing properties.

The invention further relates to a compartment module for use in an inhaler for the administration of one or more substances to the human or animal body by means of inhalation, wherein the compartment module comprises one or more compartments with substance(s) and wherein the compartment module is adapted to be arranged in extension of a mouthpiece module comprising a mouthpiece with means for perforating said compartment in said compartment module thereby allowing said substance(s) to be inhaled through the mouthpiece.

The invention further relates to a mouthpiece module for use in an inhaler for the administration of one or more substances to the human or animal body by means of inhalation, wherein the inhaler comprises a compartment module comprising one or more compartments with substance(s) and wherein the mouthpiece modules are adapted to be arranged in extension of a compartment module, the mouthpiece module comprises a mouthpiece with means for perforating said compartments modules thereby allowing said substance(s) to be inhaled through the mouthpiece.

The invention further relates to a heating module for use in an inhaler for the administration of one or more substances to the human or animal body by means of inhalation, wherein the inhaler comprises a compartment module and a mouthpiece module arranged in extension of one another and wherein the heating module is adapted to be arranged in extension of a module and wherein the heating module comprises heating means for heating the air to be inhaled through said inhaler.

General advantages of using an inhaler according to the present invention instead of cigarettes when needing nicotine are the following:
- Inhaler generates no smoke.
- It has a low production price compared to production costs for producing cigarettes.
- The inhaler covers the need for substances and avoids restlessness.
- Using an inhaler does not discolor the skin.
- There is no danger of fire.
- The inhaler has a long durability/storage life
- The inhaler could function as an alternative dose dispensing device for medicals, e.g. nasal medicine, steroids for pneumatic inhalation or muocual use.
- The inhaler could also be used without nicotine, e.g. non-smoking device for young people.
- The inhaler is hygienic to use.
- The inhaler can be used for cutback of nicotine doses.
- The inhaler looks and feels like normal cigarettes.
- The inhaler is ready-to-use and user-friendly.
- Using an inhaler according to the present invention makes it possible to adjust taste, color and temperature as desired.

The following advantages are obtained as a result of the inhaler comprising modules:
- By producing the inhaler in modules the production becomes fast and inexpensive. Further, it becomes easy to change the production into other or more containers.
- A longer storage life of the container modules as the substances do not mix or get exposed to the air.
- The inhaler gets child-resistant and tamper-proof.
- It gets user-friendly and easy-to-use.
- The inhaler can easily be designed for smoke cessation.
- The inhaler is ideal for recycling because it is easy to separate the different components.
- The specific shape of the containers makes it possible for substances to expand without leakage.

The method of producing containers as described below results in an extra thin separation wall between chambers either in one container module or between two container modules.

By using the heating module according to the present invention:
- A comfortable temperature can be obtained when inhaling, which increases the feel-good experience of smoking.
- It increases the release of the substances, thereby increasing the efficiency of the inhaler
- The specific properties of the heating module according to the present invention hold the temperature for a long period.
- Further, the present invention results in no or little obstruction of the air flow.
- The heating module is easy and fast to produce and assemble in mass production.
- It is simple for the user to perform the heating operation -> user friendly.
- The heating module can be reheated.
- The heating module simulates the routine of lighting a cigarette
- The cover or cap protects against risk of burns or fire during use or after.
- The heat indicator yields a simple visual sign of the heat and a potential risk of burn and prevents overheating.
- No extra equipment is necessary for the heating (for the metal pin or rod).
- The metal part in the heating unit can be dimensioned to obtain a heating for a desired efficiency (time, temperature, max heating of inhaling air).
- Known shape and weight of the cigarette is maintained.

### BRIEF DESCRIPTION OF THE DRAWINGS

In the following, preferred embodiments of the invention will be described referring to the figures, where
figures 1-3 show embodiments of an inhaler according to the invention when heating the heating unit just before use and during use, respectively,
figure 4 shows an exterior view of an inhaler according to the invention,
figures 5-6 show the different parts of the same inhaler as in figure 1 in a cross-sectional view and in a perspective view, respectively,
figure 7 shows another embodiment of an inhaler according to the invention,
figure 8 illustrates a container module according to one embodiment of the invention comprising two compartments for substances,
figure 9 shows in greater details a part of a cover for the container module from figure 8,
figure 10 shows different patterns which advantageously can be imprinted on the covers for a container module,
figures 11 and 12 show different types of imprinting in a cross sectional view,
figure 13 illustrates a method of manufacturing a container module according to one embodiment of the invention by injection moulding,
figures 14 shows another embodiment of a heating unit for an inhaler according to the invention,
figure 15 shows an embodiment of a partially transparent cover for a heating unit,
figure 16 shows a further embodiment of a heating unit for an inhaler,
figure 17 illustrates a heating station for heating up a heating unit in an inhaler, and
figure 18 shows the heating station from figure 17 with an inhaler according to one embodiment of the invention being heated.

### DESCRIPTION OF PREFERRED EMBODIMENTS

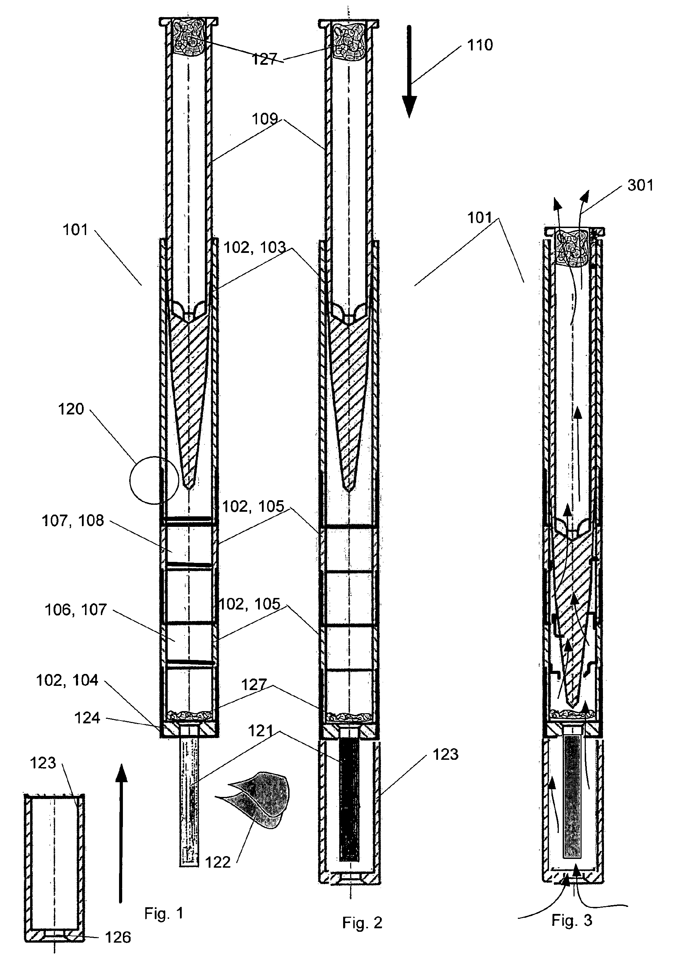

Figures 1-6 illustrate an embodiment of an inhaler 101 and the different parts of the inhaler according to the present invention shown in different situations and views.

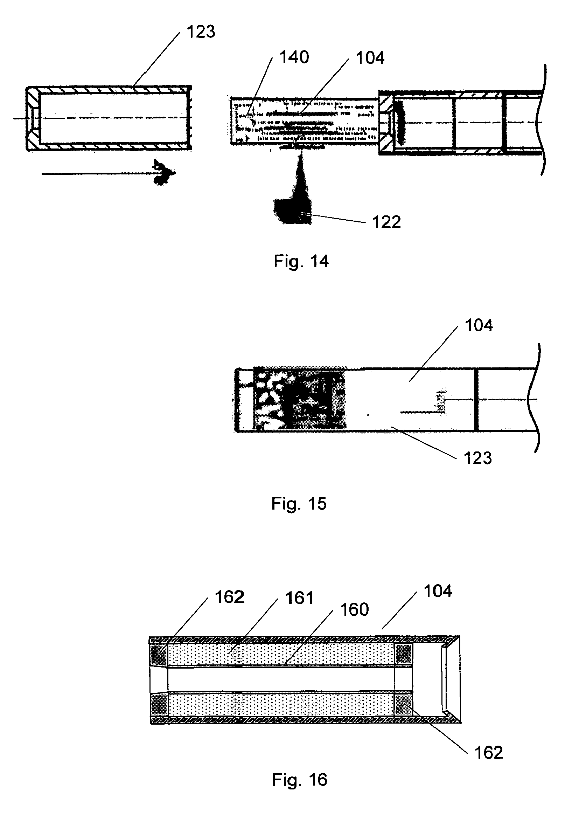

In figure 1 is shown in a cross sectional view of the inhaler 101 prior to inhalation while the heating unit is being heated. The inhaler is here built up by a number of modules 102 or units that are joined in some way. The modules can e.g., as is the case in the shown embodiment, be clicked together with a key and tongue connection 120, but could also equally well be joint by gluing, by heat welding or ultrasonic welding, etc. The one module in the first end comprises a mouthpiece 103 with a hollow piston 109 through which the user inhales the substances. The module in the second end away from the mouth of the user can in one embodiment of the invention comprise a heating unit 104 which can attain several different designs which will be described in more details below and under figures 14-16. The heating unit heats up the air prior to inhalation whereby the 'smoking' or inhalation comfort of the user is increased. Further, the heating increases the release of the substances to be inhaled. The inhaler could, however, also in another embodiment be produced and used as a 'cold' cigarette without a heating unit.

Prior to use, the substance or substances which are to be inhaled are kept in the container module or modules 105. If the inhaler is to be used as a smoke-free cigarette, i.e. for inhaling nicotine, it is advantageous to keep the nicotine substance 106 in a closed compartment 107 without exposure to the air whereby the durability of the product will be significantly longer. Further, if any aromatic substance or substances or helping agents are to be inhaled in combination with the nicotine, these aromatic substances 108 are advantageously kept in one or more compartments 107 separate from the nicotine substance 106. Such aromatic substances could be of the group of Beta damascenone, 4-oxo-beta-ionone, oxo-edulan I-II, 3-oxo-alpha-ionone, Dihydroactionodiolide, Megastigmatrienones (4 isomer), different Carotinoide derivates and the helping agents could for instance be different air humidifying agents, pH regulating fluids (such as picrin acid or ammonia), Eugenol (heat producer), catalysts, emulsifiers, Tannin (astringent) or different naturopathic drugs. The design of several separate compartments is illustrated in figures 1-4 where in this case two container modules 105 are placed one after another and next to the mouthpiece module 103. In this embodiment of the invention each container module 105 comprises one compartment 107 and one or a mix of compatible substances. Which substance 106, 108 is placed the closest to the mouthpiece module could be determined by how the substances will mix up and blend.

Prior to inhalation the heating unit 104 (if any) is heated. In this embodiment shown in figures 1-3 the heating unit comprises a pin 121 or body made of a material with good heat-conducting or -convecting properties such as cupper, brass or bronze metal alloys. The pin 121 can simply be heated by a flame 122 from e.g. a lighter or a match as illustrated in figure 1 thereby heating the air flow inhaled by the user. The pin is in the shown embodiment attached to the rest of the inhaler by means of a disk 124 which apart from acting as attachment means for the pin also acts to isolate the pin from the other modules of the inhaler. The disk is hence made of a heat insolating material such as ceramic or the like. In this embodiment the free air passage is ensured by a number of perforations in the disk (not shown). The pin in the heating unit can in a further embodiment comprise a heat indicator as known from e.g. the welding industry. The indicator can e.g. change color from grey or black to red along with the temperature of the pin thereby indicating to the user when an appropriate temperature of the heating unit is obtained. After heating a detachable cover or cap 123 can be replaced covering the metal pin 121 by the user. This prevents any risk of burns to the user from the heated pin 121 during use as well as any risk of fire after disposal. The cover 123 is manufactured from a heat resistant and isolating material such as an isolating plastic or a ceramic. The cover can also be made of a plastic with an insulating and a thermo resistant layer on the inside. Further, the cover can be partly or totally transparent whereby is obtained an illusion of the glow of a cigarette. In different embodiments the heating unit can take other shapes than a pin, e.g. as a hollow metal cylinder instead. The airflow will then go in through the interior of the cylinder and into the container modules. The use and effect of the cylinder would be the same. Yet further embodiments of the heating unit are shown in figures 14-16.

Figure 2 illustrates the situation with the pin 121 in the heating unit 104 being heated up to an appropriate temperature and the cover 123 being replaced covering the pin.

The inhaler is activated by perforating the compartment(s) with substances by pressing in a piston 109 as illustrated by the arrow 110 in figure 2. The activated and ready-to-use inhaler with the piston having perforated the end walls of the substance compartments is shown in figure 3. The piston is hollow whereby a free air passage is provided. The substances are thus allowed to mix and blend and are exposed to the air whereby the different drugs evaporate and are released for the user to inhale through the mouthpiece 103. The airflow then runs as sketched by the arrows 301 in figure 3, through the end opening 126 in the cover, gets heated while passing alongside the pin 121 in the heating unit 104, passes through the now perforated and open compartments 107 and is inhaled by the user through the hollow piston 109 in the mouthpiece module 103. If necessary, the heating of the pin in the heating unit can be repeated if for example the user wishes to resume inhalation after a break.

One or two filters 127 are placed in the inhaler. One is placed in the hollow space of the piston 109 and optionally another thin filter next to the heating unit and the last container module. The filter 127 in the piston 109 ensures that none of the substances can possible get into the mouth of the user even if the substances are not fully evaporated. The filter can e.g. be an acetate tow filter or a cellulose filter.

The different parts and modules of the inhaler can e.g. be made of different plastic materials, metal foils of e.g. Aluminum, Barex or metal alloys. Alternatively the container modules can be manufactured of a composite construction with a coating of a diffusion-tight membrane. To give the user a more realistic feeling of holding and 'smoking' a cigarette the inhaler is in one embodiment wrapped with cigarette and filter paper.

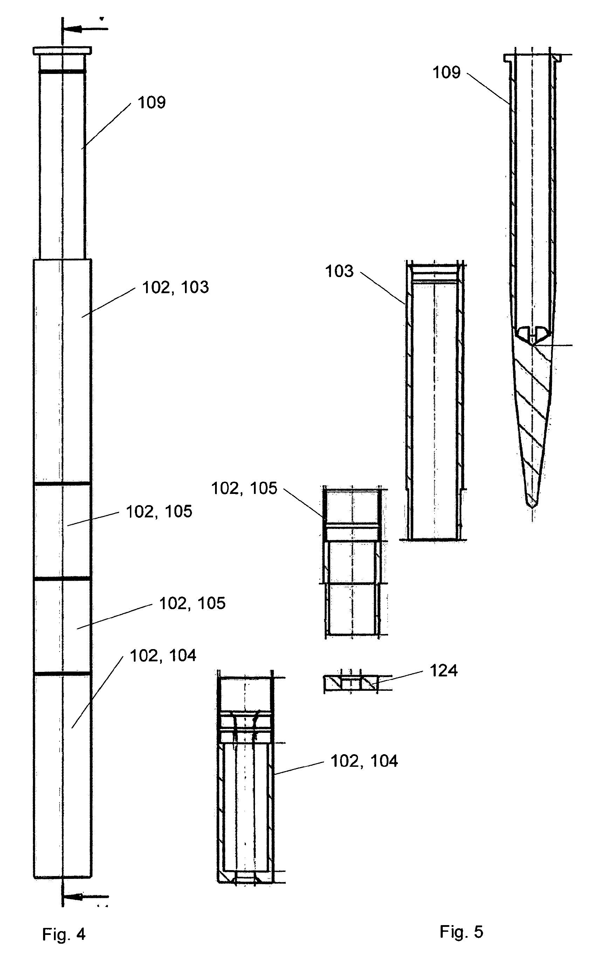

In figure 4 is shown the appearance of the inhaler 101 as it looks with the piston 109 ready to be pushed in and with the cover 123 on. Here, the different modules 102, (the mouthpiece module 103 with the piston 109, the container modules 105 and the heating module 104) can easily be seen.

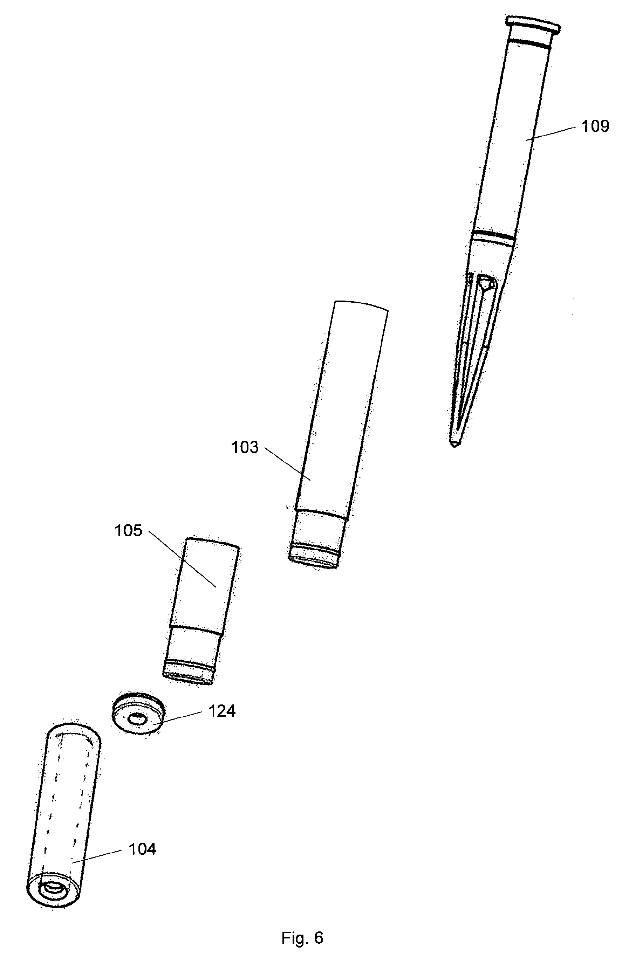

Figures 5 and 6 show the different parts or modules 102 of the inhaler 101 in a cross-sectional view and a perspective view, respectively. For clarity only one container module 105 is shown. In the embodiments of the inhaler previously shown, two container modules have been used, but the construction of modules makes it very easy production wise to manufacture an inhaler with more container modules. This could be desirable if more than two substances or mixes of substances were desired for the final blend to be inhaled, which substances would for instance last longer if kept separated in separate compartments. Further, different blends of substances could simply be obtained by stacking and assembling a number of available substances in different series or combinations.

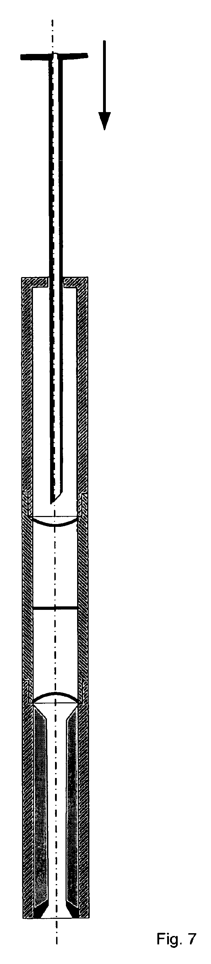

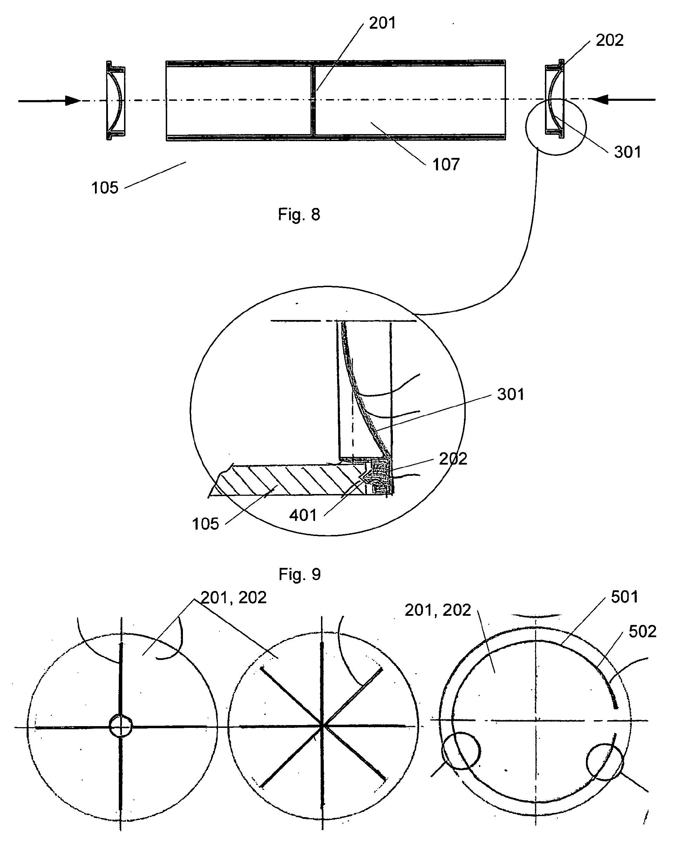

In figure 7 is shown another embodiment of the inhaler according to the invention built up by a number of modules. The modules, as in the last figures 1-6, comprise a mouthpiece module 103 and a heating module 104 in each end of the inhaler. Here, only one container module is present, but comprising two separate compartments 107 separated by a thin interior wall 201 and sealed off to the exterior by lids or covers 202 at both ends. The container module is shown in more detail in figure 8. The piston 109 for perforating the covers or lids 202 of the compartments and for allowing the substances to mix can have many different designs, its principle feature being its ability to perforate all the substance compartments 107 yielding a free passage for the substances to be inhaled through the mouthpiece 103. In figure 7 the piston 109 attains a more needle-like shape whereas it in figures 1-6 takes the shape of a cone with one or more cuts.

The modular construction of the inhaler is also advantageous for recycling purposes in that the used and disposed inhaler easily and fast can be broken apart and separated into its module parts and sorted accordingly. In this way any metals and/or plastics used in some of the modules can easily be sorted out for recycling.

When the inhaler is manufactured, the modules can firstly be produced separately. The compartment in one container module is filled with a substance or mix of substances and then either closed or sealed off directly by attaching a lid or by the attachment of the first module to its neighboring module. If this is also a container module, this is also filled with the desired substance and attached to the next module. The last container module can then in one embodiment be closed by attachment to the mouthpiece. The containers are in one embodiment welded together under an inert gas atmosphere in order to ensure leak-tight connections. Due to the modular design, the manufacture of the inhaler becomes very fast, simple and cost-effective. Furthermore, the production can very easily be adjusted to specific demands of e.g. different lengths, different combinations of substances used, different types of mouthpieces, etc. It is also straightforward and fast to change the production into making inhalers with more initially separated compartments of substances by simply adding one or more extra container modules.

A container module for substances according to one embodiment of the invention is shown in greater detail in figure 8. The module in this embodiment comprises two compartments 107 separated with an interior wall 201. The wall need not part the module in two compartments of equal size, but can equally well be placed anywhere between the ends of the module. Advantageously, the interior wall can be placed so that the volumes of the two compartments correspond to the optimal ratio of mixture of the substances in the two compartments. In one embodiment, one compartment 107 of the container module 105 is first filled with a substance (e.g. nicotine in the case of a smoke-free cigarette) under an inert atmosphere, and the compartment is closed and sealed off with a lid or cover 202. The cover can be welded to the container by, for instance, ultrasonic welding or heat sealing whereby a complete sealing without any leaks is ensured. The container module is then turned 180 degrees, and the operations are repeated with the filling of the second compartment with the other substance (for instance aromatic substances in the case of a smoke-free cigarette), closing, and sealing. Hereafter the container module is ready to be clicked onto or in other ways assembled to the other modules (such as a heating unit, a mouthpiece module and possibly more container modules) of the inhaler as for instance sketched in figures 1 and 7. The substances will in this way not be in contact with the air or exposed to e.g. the sunlight and will thus not start to break down and be released until the covers and/or the interior wall is perforated by the piston or in other ways broken. The durability of the inhaler is this way greatly lengthened. The design of the container module 105 is furthermore advantageous in facilitating an effective and fast, yet simple process for providing the substances to an inhaler.

In one embodiment the covers of the container module are designed with end faces 301 dishing inward. Hereby is obtained that it is possible for the substance within the compartment to expand according to temperature and/or pressure. This is advantageous as leaks are prevented and the durability of the product is ensured even at rather extreme temperatures and/or pressures.

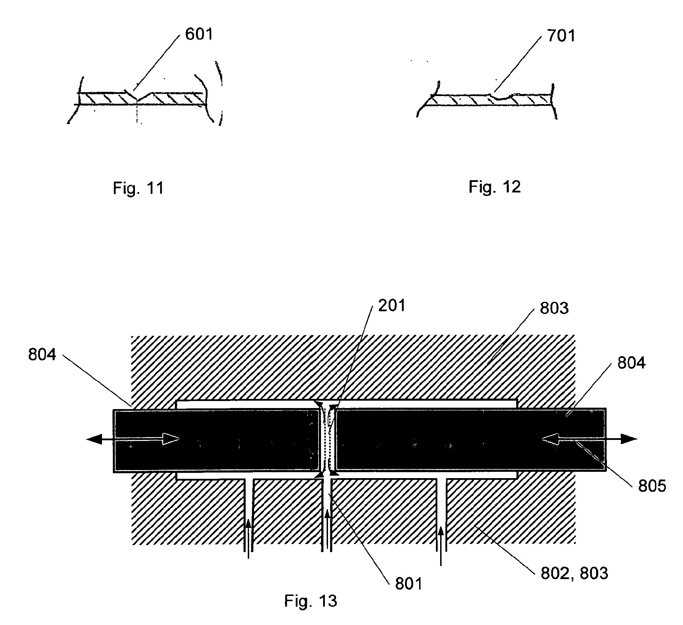

A cover 202 and one way of attaching it to a container module 105 is shown in detail in figure 9. As mentioned above, the end face arches inward towards the interior of the compartment. The cover or lid 202 is in this embodiment further provided with a convexity of weld or a bead 401 for facilitating the welding of the cover onto the module wall 105. The covers 202 as well as any interior wall 201 separating the compartment(s) filled with substance(s) are in one embodiment of the invention pre-embossed or imprinted with a pattern 501 whereby the perforation of the cover or end wall is greatly facilitated. Different imprint patterns 501 are shown in figure 10 and in figures 11 and 12 in a sectional view. As sketched in figure 10 the patterns could comprise circle sections 502 or cross or star shapes. The optimal pattern depends on the shape of the perforation means (the piston) as well as the viscosity of the different substances. One importance feature of the patterns is furthermore that it should ensure that the free air passage will not be blocked by the torn remaining parts from the separation walls or covers. As illustrated in figures 11 and 12, the indention of the pattern can also attain different indention profiles, such as a fairly sharp profile 601 or a more rounded profile 701.

A method for the manufacture of a container module such as the one shown in figure 3 is illustrated in figure 13. The container module 105 can advantageously be manufactured by plastic injection molding. The thermoplastic material is injected through one or more injection openings 801 into the mould 802. The injection openings can be placed in a number of places and not only as illustrated in the figure. The mould comprises two or more form parts 803, and two cores 804 movable in the direction of the arrows 805. When the plastic material has almost filled the mould cavity, the two cores are forced towards one another thereby pressing the excess material out into the walls of the container module and making the interior wall 201 thinner than otherwise possible with traditional manufacturing methods. After injection the cores are withdrawn, the form parts opened and the finished container module ejected from the mould.

Figures 14 and 15 show the end of an inhaler with another embodiment of a heating unit 104 with and without a protective cover or cap 123. In this embodiment the heating is achieved by the heating up of a foamed metal part 140, e.g. by a flame 122. The part has an open-celled structure whereby the inhaled air can flow through the foamed part and in this way be heated. As also mentioned earlier, the heating is advantageous mainly for two reasons: First, the user then inhales warmer air closer to or exceeding the body temperature which increases the comfort. For a smoker the inhaler is thereby also experienced more and feels more like a traditional cigarette. Secondly, the heat increases the release and evaporation of the substances in the air.

In figure 15 is illustrated the appearance of a cover or cap 123 which is partly transparent whereby the inner foamed metal part is partly visible.

In figure 16 is shown another embodiment of a module comprising a heating unit which heats and warms up the surrounding air; primarily the air flowing through the air passage. The heating unit comprises an inner tube 160 of a metal or metal alloy with good heat conducting properties such as a brass and/or Tombak brass, a cupper alloy or a bronze. The space 161 between the inner heat conducting tube 160 and the exterior wall is partly or totally filled with an accumulating material with good heat absorbing properties, which when heated accumulates the heat and releases it slowly again. This could for instance be a paraffin, Natrium, a metal salt, a PSM (phase Shift Material), or any other material especially suited for heat accumulation in the 40°-80° Celsius range. The space 161 is closed in both ends with corks or plugs 162. In one embodiment the heat loss to the exterior is minimized by isolating the module to the exterior. The exterior wall of the module can for instance be made of a foamed plastic with air voids yielding good isolating properties.

The heating unit is to be heated prior to use of the inhaler and optimally prior to pressing the piston down into the inhaler thereby activating the inhaler. The accumulating material is in one embodiment of the invention heated to approximately 55° Celsius which is an appropriate temperature to ensure heating of the air flow for a suitable period long enough for the user to finish inhaling the substances. Furthermore, a temperature of this approximate size ensures that the user will not be burned upon using the heated inhaler.

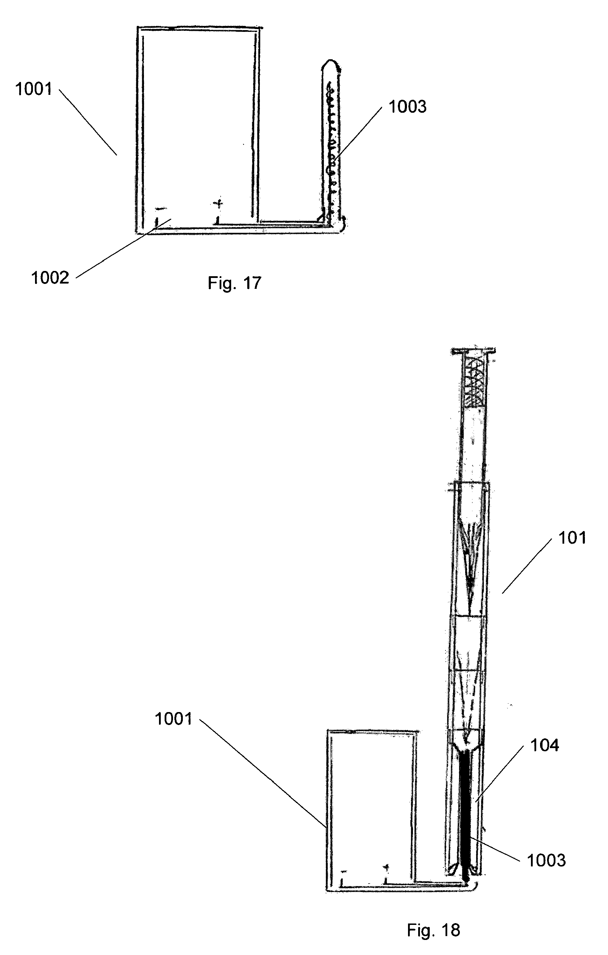

The accumulating material can be heated in a number of ways. In one embodiment the accumulating material is heated by an exterior heating station 1001 as sketched in figure 17. The heating station 1001 basically comprises an energy source 1002 such as a battery which heats a cylindrical heat conducting unit 1003.

In figure 18 is shown the inhaler as placed in the heating station 1001 during the heating of the accumulating material.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word 'comprising' does not exclude the presence of other elements or steps than those listed in a claim.

## Claims

1. An inhaler for the administration of one or more substances to the human or animal body by means of inhalation, the inhaler comprising at least two modules arranged in extension of one another of which
- one module is a compartment module comprising one or more compartments with substance(s), and
- one module is a mouthpiece module comprising a mouthpiece with means for perforating said compartments in said compartment modules thereby allowing said substance(s) to be inhaled through the mouthpiece.

2. An inhaler according to claim 1 wherein one module is a heating module comprising heating means for heating the air to be inhaled through said mouthpiece of said mouthpiece module.

3. An inhaler according to any of claims 1-2, where a compartment in one compartment module is closed by the module being in extension of said compartment module.

4. An inhaler according to any of claims 1-3, where said substances comprise nicotine and one or more aroma substances.

5. An inhaler according to any of claims 1-4, wherein the compartment module comprises two separate compartments.

6. An inhaler according to any of claims 1-5, where the compartment module comprises end faces arching inwards towards the interior of the compartment.

7. An inhaler according to any of claims 1-6, where the compartment module comprises end faces with imprint patterns for facilitating perforation of the end faces thereby the compartments.

8. An inhaler according to any of claims 1-7, wherein the perforation means of said mouthpiece module comprises a hollow piston.

9. An inhaler according to any of claims 1-8, wherein the mouthpiece module further comprises a filter.

10. An inhaler according to any of claims 2-9, wherein said heating means are made of a material which has good heat-convecting or heat-conducting properties, such as cupper, brass or bronze adapted to be heated by an external heat source.

11. An inhaler according to any of claims 2-10, wherein said heating means are pin shaped.

12. An inhaler according to any of claims 2-10, wherein said heating means are shaped like a hollow cylinder adapted to receive a pin shaped heating source.

13. An inhaler according to any of claims 2-12, wherein the heating means are made from foamed metal.

14. An inhaler according to any of claim 2-13, wherein the heating means comprises a heat indicator.

15. An inhaler according to any of claims 2-14, wherein the heating module comprises isolation means for heat isolating the heating means from other modules of the inhaler.

16. An inhaler according to any of claims 2-15, wherein the heating module comprises a detachable cover for covering the heating means.

17. An inhaler according to any of claims 2-16, wherein the heating means are chamber and at least partly filled with an accumulating material having good heat absorbing properties.

18. A compartment module for use in an inhaler for the administration of one or more substances to the human or animal body by means of inhalation, wherein the compartment module comprises one or more compartments with substance(s) and wherein the compartment module is adapted to be arranged in extension of a mouthpiece module comprising a mouthpiece with means for perforating said compartment in said compartment module thereby allowing said substance(s) to be inhaled through the mouthpiece.

19. A compartment module according to claim 18, wherein the compartment module comprises two separate compartments.

20. A compartment module according to any of claims 18-19, where said substances comprise nicotine and one or more aroma substances.

21. A compartment module according to claims 18-20, where the module comprises end faces arching inwards towards the interior of the compartment.

22. A compartment module according to claims 18-21, where the module comprises end faces with imprint patterns for facilitating perforation of the end faces thereby the compartments.

23. A mouthpiece module for use in an inhaler for the administration of one or more substances to the human or animal body by means of inhalation, wherein the inhaler comprises a compartment module comprising one or more compartments with substance(s) and wherein the mouthpiece module is adapted to be arranged in extension of a compartment module, the mouthpiece module comprises a mouthpiece with means for perforating said compartment modules thereby allowing said substance(s) to be inhaled through the mouthpiece.

24. A mouthpiece module according to claim 23, where said perforation means comprises a hollow piston.

25. A mouthpiece module according to any of claims 23-24, wherein the module further comprises a filter.

26. A heating module for use in an inhaler for the administration of one or more substances to the human or animal body by means of inhalation, wherein the inhaler comprises a compartment module and a mouthpiece module arranged in extension of one another and wherein the heating module is adapted to be arranged in extension of a module and wherein the heating module comprises heating means for heating the air to be inhaled through said inhaler.

27. A heating module according to claim 26, wherein said heating means are made of a material which have good heat-convecting or heat-conducting properties, such as cupper, brass or bronze adapted to be heated by an external heat source.

28. A heating module according to any of claims 26-27, wherein said heating means are pin shaped.

29. A heating module according to claims 26-27, wherein said heating means are shaped like a hollow cylinder adapted to receive a pin shaped heating source.

30. A heating module according to any of claims 26-29, wherein the heating means are made from foamed metal.

31. A heating module according to any of claims 26-30, wherein the heating means comprise a heat indicator.

32. A heating module according to any of claims 26-31, wherein the heating module comprises isolation means for heat isolating the heating means from other modules of the inhaler.

33. A heating module according to any of claims 26-32, wherein the heating module comprises a detachable cover for covering the heating means.

34. A heating module according to any of claims 26-33, wherein the heating means are chamber filled with an accumulating material having good heat absorbing properties.
